## Europäisches Patentamt

## European Patent Office

## Office européen des brevets

(11) Veröffentlichungsnummer: **0 062 282**
B1

(12)

# EUROPÄISCHE PATENTSCHRIFT

(45) Veröffentlichungstag der Patentschrift:
**12.09.84**

(51) Int. Cl.³: **C 07 C 47/02**, C 07 C 45/50,
C 07 C 45/82, C 07 C 45/81

(21) Anmeldenummer: **82102664.8**

(22) Anmeldetag: **30.03.82**

(54) **Verfahren zur Gewinnung von Aldehyden.**

(30) Priorität: **08.04.81 DE 3114147**

(43) Veröffentlichungstag der Anmeldung:
**13.10.82 Patentblatt 82/41**

(45) Bekanntmachung des Hinweises auf die Patenterteilung:
**12.09.84 Patentblatt 84/37**

(84) Benannte Vertragsstaaten:
**AT BE DE FR GB IT NL**

(56) Entgegenhaltungen:
**EP - A - 0 016 285**
**DE - A - 2 715 685**

**CHEMICAL ENGINEERING, Dezember 1977, Seiten 110-115, "Low-pressure oxo process yields a better products mix"**

(73) Patentinhaber: **BASF Aktiengesellschaft,
Carl-Bosch-Strasse 38, D-6700 Ludwigshafen (DE)**

(72) Erfinder: **Fischer, Karl, Dr., Luginsland 22, D-6520 Worms
(DE)**
Erfinder: **Grenacher, Armin Volker, Dr., An der
steinernen Bruecke 10, D-6704 Mutterstadt (DE)**
Erfinder: **Herr, Manfred, Silvanerweg 6,
D-6706 Wachenheim (DE)**
Erfinder: **Strohmeyer, Max, Dr., Woogstrasse 41,
D-6703 Limburgerhof (DE)**

## Beschreibung

Die vorliegende Erfindung betrifft ein verbessertes Verfahren zur Gewinnung von Aldehyden, die bei der Hydroformylierung von olefinisch ungesättigten Verbindungen mittels Rhodium-Katalysatoren dampfförmig im Gemisch mit sonstigen Reaktionsprodukten sowie mit nicht umgesetzten Einsatzstoffen anfallen.

Die Hydroformylierung olefinisch ungesättigter Verbindungen mittels Rhodium-Katalysatoren ist allgemein bekannt (vgl. z.B. Chemical Engineering, December 1977, S. 110 ff.) und bedarf daher keiner weiteren Erläuterung, zumal sich die Erfindung nicht auf die Hydroformylierungsreaktion selber, sondern auf die Gewinnung der Aldehyde aus dem Hydroformylierungsgemisch bezieht.

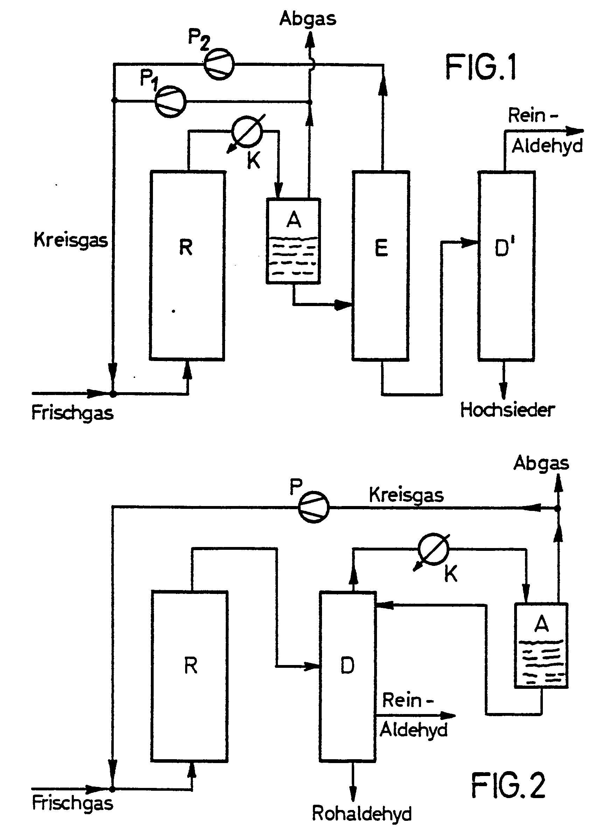

Bisher (vgl. Chem. Eng. oben) wurden die Aldehyde auf die Weise gewonnen, dass man sie zusammen mit anderen gasförmigen Bestandteilen dampfförmig aus dem Reaktor R (siehe Fig. 1) austrägt und dieses Gasgemisch im Kühler K soweit abkühlt, dass sich die Aldehyde weitgehend verflüssigen. Dieses Gemisch wird sodann im Abscheider A in eine Gasphase aus nicht umgesetztem Olefin, dem entsprechenden Paraffin, CO und $H_2$ einerseits und die flüssige Aldehydphase andererseits, getrennt, wonach die Gasphase grösstenteils komprimiert und wieder als sogenanntes Kreisgas zusammen mit frischen Einsatzstoffen in die Hydroformylierungsstufe zurückgeführt wird. Damit die Menge der nicht verwertbaren Gase wie Paraffin und Stickstoff nicht ständig zunimmt, muss ein Teil des Kreisgases als Abgas aus dem System entfernt werden.

Da die in A anfallende Flüssigphase noch erhebliche Mengen des Olefins gelöst enthält, ist es zur Vermeidung von Olefinverlusten erforderlich, diese Gase durch erneutes Erhitzen in der Entgasungskolonne E aus der Aldehydphase auszutreiben. Die hierbei gewonnenen Gase werden anschliessend wieder komprimiert und danach als Kreisgas-Teilstrom mit dem erstgenannten Kreisgas-Strom vereinigt und somit ebenfalls wieder in die Synthesestufe zurückgeführt. Die in E anfallende Flüssigphase wird sodann wie üblich destillativ aufgearbeitet, etwa indem man sie in einer Kolonne D' in die Aldehydfraktion und hochsiedende Sumpfprodukte zerlegt, wonach sich noch weitere Aufarbeitungsschritte anschliessen können.

Sieht man von den Druckverlusten ab, welche durch die Kompressoren $P_1$ und $P_2$ ausgeglichen werden, steht das System einschliesslich der Entgasungskolonne E unter dem für die Hydroformylierung gewählten Druck von etwa 1 bis 40 bar. Die Entspannung auf Normaldruck kann nach der Kolonne E oder an einer sonstigen Stelle des weiteren Verfahrensablaufs vorgenommen werden.

Nachteilig bei dieser Arbeitsweise ist indes, dass man die Gesamtmenge des Rohaldehyds zunächst durch Kühlung kondensieren und danach wieder in der Entgasungskolonne erhitzen muss.

Der Erfindung lag daher die Aufgabe zugrunde, den hierdurch bedingten Energieverbrauch zu reduzieren. Ausserdem war es Aufgabe der Erfindung, die Gewinnung der Aldehyde apparativ zu vereinfachen.

Demgemäss wurde ein Verfahren zur Gewinnung von Aldehyden aus dem gasförmigen Austrag der Hydroformylierung von olefinisch ungesättigten Verbindungen mittels Rhodium-Katalysatoren gefunden, welches dadurch gekennzeichnet ist, dass man

a) diesen Austrag ohne zu kühlen und ohne zu entspannen in eine Destillationskolonne D einführt,

b) die Kopffraktion dieser Kolonne in einem Kühler K so weit kühlt, dass der überwiegende Teil der hierin enthaltenen Aldehyde kondensiert,

c) das Kondensat in einem Abscheider A in eine Gasphase und eine Flüssigphase trennt,

d) die Gasphase von A nach Abtrennung des Abgases mittels eines Kompressors P wieder auf Synthesedruck bringt und als Kreisgas in den Reaktor zurückführt,

e) die Flüssigphase von A nach D zurückführt, und

f) die Aldehyde der Kolonne D als flüssigen Sumpfabzug und/oder dampfförmigen Seitenabzug entnimmt.

Fig. 2 zeigt das Schema dieses Verfahrens, nach welchem sich vom Ausgang des Reaktors bis zur Stufe des von den Gasen befreiten Aldehyds etwa 30% Energie einsparen lassen, verglichen mit der bisherigen Arbeitsweise.

Das erfindungsgemässe Verfahren eignet sich für die Gewinnung von all denjenigen Aldehyden, deren Partialdruck so gross ist, dass sich ein dampfförmiger Austrag lohnt. Dies trifft vor allem für die Herstellung von Propionaldehyd aus Ethylen, von Butyraldehyd aus Propylen und allgemein bis zu den $C_5$-Aldehyden aus den entsprechenden $C_4$-Olefinen zu. Besondere Bedeutung hat das Verfahren für die Gewinnung von Propionaldehyd und der Butyraldehyde.

Die Hydroformylierung wird wie üblich bei etwa 1 bis 40 bar vorgenommen. Demgemäss stehen auch alle Anlagenteile bis zur Stelle der Produktentnahme von der Kolonne D praktisch unter diesem Druck. Der unvermeidbare Druckverlust infolge von Leitungswiderständen beträgt etwa 2 bar und wird durch den Kreisgaskompressor P kompensiert.

Die Anzahl der theoretischen Böden in der Kolonne D ist weitgehend unabhängig von der Art der Hydroformylierungsprodukte und beträgt etwa 4 bis 12. Die Temperaturen am Kopf der Kolonne D entsprechen den Hydroformylierungstemperaturen, die bei den niederen Olefinen etwa zwischen 50 und 140°C liegen.

Auf die Bauart der Kolonne D kommt es nicht an, so dass man hier zweckmässigerweise in der Regel die einfachen Füllkörperkolonnen verwendet.

Zur Kondensation der Hydroformylierungsprodukte im Kühler K reicht im Falle des Ethylens und des Propylens eine Kühlung auf etwa 50 bis 70°C aus.

Die Gase, vornehmlich die olefinisch ungesättigten Verbindungen, die entsprechenden gesättigten Verbindungen und geringe Mengen der Verfahrensprodukte werden grösstenteils über die Pumpe P als Kreisgas wieder in den Reaktor zurückgeführt. Ein kleiner Teil der Gase, etwa 1 bis 5 Vol.% wird als Abgas aus dem System entfernt.

Der Rohaldehyd kann der Kolonne D als flüssiges Sumpfprodukt entnommen werden, jedoch empfiehlt es sich besonders, einen Teil des Aldehyds — etwa 40 bis 80% — als dampfförmigen Reinaldehyd-Seitenabzug abzuführen, weil sich hierdurch der Aufwand für die weitere Reinigung des Rohaldehyds verringert. Ferner kann es zweckmässig sein, den gesamten Rohaldehyd als dampfförmigen Seitenabzug zu entnehmen, so dass als Sumpfprodukt in der Kolonne D nur die hochsiedenden Rückstände verbleiben. Im übrigen kann der Rohaldehyd wie üblich weiter destillativ gereinigt werden.

*Beispiel*

In einem Versuchsreaktor R (siehe Fig. 2) wurden stündlich 15 kg Ethylen mittels eines Rhodium-Katalysators bei 110°C und 16 bar wie üblich hydroformyliert. Der gasförmige Reaktoraustrag wurde ohne Kühlung und Entspannung auf Höhe des 6. Bodens einer Füllkörperkolonne D mit insgesamt 8 theoretischen Böden zugeführt und dort fraktioniert.

Die Kopffraktion der Kolonne D wurde im Kühler K von 90 auf 40°C gekühlt, wonach die verflüssigten Anteile im Abscheider A von der Gasphase getrennt und auf den Kopf der Kolonne D zurückgeführt wurden.

Von der Gasphase, die im wesentlichen aus CO, $H_2$, Ethylen und Ethan bestand, wurden rund 0,9 kg/h als Abgas ausgeschleust. Der übrige Teil (rund 31,1 kg/h) wurde über den Kompressor P (Druckdifferenz rund 2 bar) als Kreisgas zusammen mit Frischgas wieder in den Reaktor R zurückgeführt.

Am Sumpf der Kolonne D fielen bei 153°C stündlich 29,7 kg roher Propionaldehyd an. Dieser Rohaldehyd enthielt noch rund 0,1 Gew.-% Ethylen sowie rund 1,1 Gew.-% eines Gemisches aus Propanol und höhersiedenden Nebenprodukten.

In einer abgewandelten Verfahrensweise wurden der Kolonne D auf Höhe des 2. Bodens stündlich 19,1 kg Propionaldehyd dampfförmig entnommen, der eine Reinheit von 99,8% hatte. Die restliche Menge des Verfahrensproduktes fiel als Rohaldehyd flüssig im Sumpf der Kolonne bei 157°C an.

**Patentanspruch**

Verfahren zur Gewinnung von Aldehyden aus dem gasförmigen Austrag der Hydroformylierung von olefinisch ungesättigten Verbindungen mittels Rhodium-Katalysatoren, dadurch gekennzeichnet, dass man

a) diesen Austrag ohne zu kühlen und ohne zu entspannen in eine Destillationskolonne D einführt,

b) die Kopffraktion dieser Kolonne in einem Kühler K so weit kühlt, dass der überwiegende Teil der hierin enthaltenen Aldehyde kondensiert,

c) das Kondensat in einem Abscheider A in eine Gasphase und eine Flüssigphase trennt,

d) die Gasphase von A nach Abtrennung des Abgases und nach Kompression mittels eines Kompressors P wieder auf Synthesedruck bringt und als Kreisgas in den Reaktor zurückführt,

e) die Flüssigphase von A nach D zurückführt, und

f) die Aldehyde der Kolonne D als flüssigen Sumpfabzug und/oder dampfförmigen Seitenabzug entnimmt.

**Claim**

A process for obtaining an aldehyde from the gaseous product of the hydroformylation of an olefinically unsaturated compound using a rhodium catalyst, wherein

a) this gaseous product is introduced, without being cooled or let down, into a distillation column D,

b) the overhead fraction from this column is cooled in a condenser K to such an extent that the greater part of the aldehydes contained therein is condensed,

c) the condensate is separated, in a separator A, into a gas phase and a liquid phase,

d) the gas phase from A, after waste gas has been separated off, is again brought to the synthesis pressure by means of a compressor P, and is returned to the reactor as a recycle gas,

e) the liquid phase from A is returned to D, and

f) the aldehydes are taken off from column D as liquid bottoms products and/or as a vaporous side stream.

**Revendication**

Procédé pour isoler les aldéhydes du produit gazeux sortant de l'hydroformylation de composés à insaturation oléfinique sur des catalyseurs au rhodium, caractérisé en ce que:

a) on envoie ce produit sortant, sans le refroidir ni le détendre, dans une colonne à distiller (D),

b) on refroidit la fraction de tête de cette colonne dans un réfrigérateur (K) dans une mesure suffisante pour condenser une proportion prépondérante des aldéhydes contenus,

c) on sépare le condensat dans un séparateur (A) en une phase gazeuse et une phase liquide,

d) après séparation des gaz résiduaires et après recompression à la pression de synthèse à l'aide d'un compresseur (P), on renvoie la phase gazeuse de A, en tant que gaz de recyclage, dans le réacteur,

e) on recycle la phase liquide de A à D, et

f) on prélève les aldéhydes de la colonne (D) en pied de colonne à l'état liquide et/ou en prélèvement latéral à l'état de vapeur.

0 062 282

FIG.1

FIG.2

5